# EUROPEAN PATENT APPLICATION

(11) **EP 4 806 432 A1**
(43) Date of publication of application: **16.09.2026**
(21) Application number: 25163737.7
(22) Date of filing: 14.03.2025
(51) Int. Cl.: A61K 31/225, A61K 39/00, A61K 45/06, A61P 29/00, A61P 37/00, A61P 37/02

(54) **COMBINATION OF IMMUNOMODULATORY AGENT AND TREG ACTIVATOR FOR THE TREATMENT OF AN AUTOIMMUNE AND AUTOINFLAMMATORY DISEASE**

(71) Applicant: ActiTrexx GmbH, 55131 Mainz (DE)
(72) Inventor: Schneider, Franz-Joseph, 1120 Wien (AT); Jonuleit, Helmut, 65428 Rüsselsheim (DE); Schlöder, Janine, 55118 Mainz (DE)
(74) Representative: Patentanwälte Dr. Keller, Schwertfeger Partnerschaft mbB

(57) **Abstract**

The present invention relates to an immunologically effective combination and a pharmaceutical composition, comprising at least one immunomodulatory agent and an immunoregulatory agent for use in the prevention or treatment of an autoinflammatory disease. The invention also relates to the use of an immunologically effective combination for the manufacturing of a medicament that promotes and restores immune tolerance in a human or animal subject.

## Description

The present invention relates to immunologically effective combinations, comprising at least one immunomodulatory agent and a CD4-targeting activator of regulatory T cells (Treg activator) as an immunoregulatory agent for use in the prevention or treatment of an autoimmune disease or autoinflammatory disease. Furthermore, the invention relates to pharmaceutical compositions, comprising an immunologically effective combination and the use of such pharmaceutical compositions in the prevention or treatment of an autoimmune or autoinflammatory disease.

Autoimmune and autoinflammatory diseases represent a broad and heterogeneous group of disorders characterized by an aberrant immune response in which the body's defense system mistakenly targets its own tissues. This loss of immune tolerance results in chronic inflammation and progressive tissue damage that can affect virtually any organ system. Common autoimmune and autoinflammatory diseases include rheumatoid arthritis, systemic lupus erythematosus, diabetes type 1, multiple sclerosis, inflammatory bowel diseases, ulcerative colitis, psoriasis, and many others. Although the specific pathology varies from joint destruction in rheumatoid arthritis to demyelination in multiple sclerosis, the underlying mechanism generally involves complex interactions between innate and adaptive immune responses.

Central to these conditions is an immune dysregulation and the breakdown of self-tolerance, where autoreactive lymphocytes become activated. This leads to the production of autoantibodies and /or the release of inflammatory cytokines (e.g., TNF-alpha, IL-6, IL-17) that perpetuate inflammation and drive tissue injury. Some autoimmune diseases target specific organs such as the pancreas in diabetes type 1 or the myelin sheath in multiple sclerosis, while others, like systemic lupus erythematosus, involve multiple organs simultaneously.

Although autoimmune and autoinflammatory diseases differ in their primary trigger, local environment and pathologies, all diseases lead to similar immunological cascades with similar signaling pathways and autoreactive lymphocytes. These components get activated and regulated to varying degrees depending on its underlying autoimmune trigger, the tissue localization and inflammatory environment.

The management of autoimmune and autoinflammatory diseases typically involves a combination of symptomatic relief and long-term immunomodulation with small molecules and biologics, called immunosuppressive drugs, disease-modifying drugs, immunomodulating drugs or anti-inflammatory drugs. However, not all patients respond adequately to available treatments, and some may experience relapses or significant side effects.

US 10,729,742 B2 describes a method of treating an autoimmune inflammatory disease using HIV-1 glycoprotein gp120 (GP120) as Treg cell activator. However, the problem of autoimmune-mediated reactivity and inflammation has not been addressed and therefore patients still suffer of autoimmune responses. Clinical data that are available so far show that blocking just one signaling pathway or inflammatory mediator is not enough to effectively control and stop inflammation in autoimmune diseases in all patients. And even combinations of anti-inflammatory drugs, immune modulatory drugs and disease-modifying drugs targeting different signaling pathways show no significant improvement to a monotherapy.

New strategies to attenuate autoimmune-mediated reactivity and inflammation are urgently needed to decrease and switch off the inflammatory response and to restore immune tolerance without comprehensively suppressing general immunity.

Against this background, it is the object of the present invention to provide novel compositions and methods for the prevention or treatment of autoimmune or autoinflammatory diseases that are able to modulate, suppress or modify immune responses in a subject without specifically targeting immune tolerance mechanisms, and to promote and restore immune tolerance by activating Treg and thereby suppressing autoinflammatory T effector cells.

This object is solved by an immunologically effective combination, comprising at least one immunomodulatory agent and an CD4-targeting activator of regulatory T cells (Treg activator).

The present invention is based on the surprising finding that a combination of an immunomodulatory agent and a Treg activator such as an anti-CD4 antibody or GP120 leads to attenuated inflammation, while a Treg activating activity downregulates autoimmune T cell response. As shown herein, these underlying mechanisms apply to any immunomodulatory agent that modulates, suppresses or modifies immune responses in a subject without specifically targeting immune tolerance. Based on these principles, the inventive immunologically effective combination provides a novel therapeutic approach and is thus suitable in the treatment or prevention of a variety of autoimmune and autoinflammatory diseases, either by administering a therapeutically effective amount or pharmaceutically effective amount of at least one immunomodulatory agent prior to or concurrently with at least one Treg activator to a subject in need thereof. The administering may be repeated as necessary or desired to result in a desired level of attenuated inflammation and autoimmune T cell response.

A subject in need thereof refers to any subject or individual who could benefit from the administration of an immunologically effective combination or method of treatment described herein. In certain embodiments, a subject in need thereof is a subject predisposed for the development of an autoimmune or autoinflammatory disease; a subject having one or more disorders related to an autoimmune or autoinflammatory disease but not exhibiting any clinical symptoms; and/or a subject exhibiting symptoms of an autoimmune or autoinflammatory disease. In one aspect "the subject in need thereof" refers to a vertebrate, such as a mammal. Mammals include, but are not limited to, humans, other primates, rodents (i.e., mice, rats, and hamsters), farm animals, sport animals and pets. In one embodiment, the subject is a mammal such as a human. In certain embodiments, the combinations and methods find use in experimental animals, in veterinary application, and/or in the development of animal models for disease.

A "therapeutically effective amount" or "pharmaceutically effective amount" means the amount of an immunologically effective combination that, when administered to a subject for treating an autoimmune or autoinflammatory disease, is sufficient to affect such treatment. Thus a "therapeutically effective amount" is an amount indicated for treatment while not exceeding an amount which may cause significant adverse effects. The "therapeutically effective amount" will vary depending on the specific immunologically effective combination, and will also be determined by physical and physiological factors such the age, body weight, and/or clinical history of the subject to be treated. Methods for evaluating the effectiveness of therapeutic treatments are known to those of skill in the art.

"An immunologically effective combination" means a single composition containing at least one immunomodulatory agent and at least one Treg activator, or two distinct compositions, wherein the first composition contains at least one immunomodulatory agent, and the second composition contains at least one Treg activator. The compositions are configured for administering to a subject in need thereof a therapeutically effective amount or a pharmaceutically effective amount of the at least one immunomodulatory agent prior to or concurrently with at least one Treg activator. The immunologically effective combination can be a combination preparation or combination product of an immunomodulatory agent and a Treg activator as immunoregulatory agent.

The immunomodulatory agents as used in the context of the present invention are characterized in that they target signalling pathways in T cells and Treg only. Consequently, autoimmune indications und autoinflammatory diseases can be treated which are either T cell mediated or where T cells are involved in pathogenesis.

All immunomodulatory agents of the present invention have the ability to reduce the activity of pro-inflammatory cytokines, to decrease the activation and proliferation of autoreactive immune cells, and to limit the release of mediators that drive inflammation and tissue damage.

The immunomodulatory agents of the present invention contribute to a reduced inflammation, a reduced immune-mediated tissue injury and the modification of the progression of immune-driven diseases. They do not focus on enhancing or restoring regulatory immune tolerance-promoting mechanisms. This is in contrast to immunoregulatory agents that specifically boost regulatory pathways controlled by Treg and allow to restore immune tolerance.

The inventive combination enables to rebalance a dysregulated network of immune and inflammatory signals in autoimmune diseases, thereby resolving autoimmune responses and re-establishing immune-inflammatory homeostasis and immune tolerance.

By using a combination therapy, a broader mechanistic coverage is achieved which benefits treatment. The combination targets biological pathways and mechanisms of disease processes that a single agent does not fully manage. The combined, synergistic effect of the combination of an immunomodulator and a Treg activator is greater than the sum of their individual effects. This synergy will improve treatment outcomes, particularly in complex autoimmune diseases where multiple pathways are involved. A monotherapy may only target one specific component of a disease pathway. However, many autoimmune and autoinflammatory diseases involve multiple pathways or redundant systems that can bypass a single blockade. The therapeutic application of the combination according to the present invention can cover a wider range of these processes because distinct drugs are used with different mechanisms and modes of action.

In a first preferred embodiment, the immunomodulatory agent modulates, suppresses or modifies immune responses in a subject without specifically targeting immune tolerance mechanisms.

The invention covers immunomodulatory agents that uses pharmaceutical substances or active pharmaceutical ingredients falling under the International Nonproprietary Names (INN). INN facilitate the identification of pharmaceutical substances or active pharmaceutical ingredients. Each INN is a unique name that is globally recognized and is public property. A nonproprietary name is also known as a generic name. Examples of INN used in the present invention include, but are not limited to Tocilizumab, Sarilumab, (IL6R inhibitors), Ixekizumab, Secukinumab, Bimekizumab, Brodalumab (IL17 inhibitors), Risankizumab, Ustekinumab, Guselkumab (IL-23 inhibitors), Adalimumab, Etanercept, Infliximab (TNF-alpha inhibitors), Nerandomilast, Apremilast, or Roflumilast (PDE4 inhibitors), Rituximab, Ocrelizumab, Ublituximab, Ofatumumab (CD20 inhibitors), Abatacept (CD80/86 inhibitor), Vedolizumab (integrin blocker), Figolimod, Ozanimod, Ponesimod, Siponimod (sphingosine 1-phosphate receptor modulator), Alemtuzumab (lymphocyte-depleting agent), Frexalimab, Dazodalibep (CD40L blocker), Vidofludimus Calcium (pyrimidine synthesis inhibitor), Aldesleukin (IL-2), Belimumab (B cell inhibitor).

In preferred variants, the immunomodulatory agent is selected from the group consisting of IL-6R inhibitors, IL-17 inhibitors, IL-23 inhibitors, TNF-alpha inhibitors, PDE4 inhibitors, interferon, fumarate immunomodulators.

In alternative embodiments, said immunomodulatory agent is selected from the group consisting of methotrexate, retinoids (e.g. Acitretin), IL-2, CD20 inhibitors, CD80/86 inhibitors, hydroxychloroquine, integrin blockers, sphingosine 1-phosphate receptor modulator, lymphocyte-depleting agents, glatiramer acetate, CD40 inhibitors, preferably CD40L blockers, pyrimidine synthesis inhibitors, B cell inhibitors.

In some embodiments, the IL-6R inhibitor is an anti-IL6R antibody.

In some embodiments, the IL-17 inhibitor is an anti-IL17R antibody.

In some embodiments, the IL-23 inhibitor is an anti-IL23R antibody.

In some embodiments, the TNF-alpha inhibitor is an anti-TNF-alpha antibody or Etanercept.

In some embodiments, the PDE4 inhibitor is and PDE4B inhibitor.

In some embodiments, the interferon is interferon-beta-1a or interferon-beta-1b.

In some embodiments, the fumarate immunomodulator is dimethyl fumarate (DMF), diroximel fumarate or monomethyl fumarate.

In further preferred embodiments, the IL-6R inhibitor is Tocilizumab or Sarilumab

In further preferred embodiments, the IL-17 inhibitor is Ixekizumab, Secukinumab, Bimekizumab, or Brodalumab.

In further preferred embodiments, the IL-23 inhibitor is Risankizumab, Ustekinumab, or Guselkumab.

In further preferred embodiments, the TNF-alpha inhibitor is Adalimumab, Etanercept, or Infliximab.

In further preferred embodiments, the PDE4 inhibitor is Nerandomilast, Apremilast, or Roflumilast.

In further preferred embodiments, the interferon is interferon-beta-1a.

In further preferred embodiments, the fumarate immunomodulator is DMF.

In alternative embodiments, the CD20 inhibitor is Rituximab, Ocrelizumab, Ublituximab, or Ofatumumab.

In alternative embodiments, the CD80/86 inhibitor is Abatacept.

In alternative embodiments, the integrin blocker is Vedolizumab.

In alternative embodiments, the sphingosine 1-phosphate receptor modulator is Figolimod, Ozanimod, Ponesimod, or Siponimod.

In alternative embodiments, the lymphocyte-depleting agent is Alemtuzumab.

In alternative embodiments, the CD40L blocker is Frexalimab or Dazodalibep.

In alternative embodiments, the pyrimidine synthesis inhibitor is Vidofludimus Calcium.

In alternative embodiments, the IL-2 is Aldesleukin.

In alternative embodiments, the B cell inhibitor is Belimumab.

A Treg activator of the present invention is an immunoregulatory agent that promotes and restores immune tolerance by activating Treg and thereby suppressing autoinflammatory T effector cells. In a preferred embodiment, the Treg activator is an anti-CD4 antibody or a functional fragment thereof. In alternative embodiments, the Treg activator is GP120 or a biologically active fragment thereof. A biologically active fragment of GP120 has the ability to bind to its respective CD4 binding site and to promote Treg activation. The invention also comprises polypeptides or proteins bearing the CD4 binding sites of GP120. The invention furthermore comprises variants, mutants and modified versions of GP120.

Functional fragments of anti-CD4 antibodies are a part of an anti-CD4 antibody comprising a heavy chain polypeptide and/or light chain polypeptide having part or all of the binding activity as the antibody from which the functional fragment is derived. The antibody of the present invention preferably comprises both the heavy chain variable region and the light chain variable region. Examples of the functional fragment include Fd, Fv, Fab, F(ab'), F(ab)2, F(ab')2, single chain Fv (scFv), a diabody, a triabody, a tetrabody, and a minibody. Preferably, the antibody fragment is a Fab fragment. The antibodies or fragments thereof have the ability to bind to a CD4 epitope on T cells, wherein binding of the antibody or fragment thereof results in an activation of regulatory T cells. The antibody of the present invention is preferably derived from a vertebrate such as a human, mouse, rat, cow, rabbit, goat, sheep, and guinea pig. In a preferred embodiment, the anti-CD4 antibody is a humanized monoclonal antibody.

In a further preferred embodiment, the anti-CD4 antibody is Tregalizumab or Palixizumab.

The immunologically effective combination of the invention is also suitable to be used in a method of treatment of an autoinflammatory disease. In a first aspect, the at least one immunomodulatory agent is provided in a first dosage form and the Treg activator is provided as a second dosage form, wherein a dosage regimen provides for the first dosage form to be administered to a subject simultaneously with or prior to the second dosage form.

The present invention also comprises the administration of more than one immunomodulatory agent as described herein. Preferably, the autoinflammatory disease is any one of psoriasis, rheumatoid arthritis, Sjögren's syndrome, diabetes type-1, Crohn's disease and ulcerative colitis, multiple sclerosis, psoriatic arthritis, systemic lupus erythematosus (SLE), uveitis, systemic sclerosis, neuro sarcoidosis.

In preferred embodiments, for the treatment of
a. psoriasis, the immunomodulatory agent is any one of TNF-alpha inhibitor, IL-17 inhibitor, PDE4 inhibitor, interferon, or fumarate immunomodulator;
b. multiple sclerosis, the immunomodulatory agent is any one of fumarate immunomodulator, TNF-alpha inhibitor, IL-6R inhibitor, IL-17 inhibitor, PDE4 inhibitor, or interferon;
c. systemic sclerosis, the immunomodulatory agent is any one TNF-alpha inhibitor, IL-6R inhibitor, or PDE4 inhibitor;
d. rheumatoid arthritis, the immunomodulatory agent is any one TNF-alpha inhibitor, IL-6R inhibitor, or PDE4 inhibitor.
e. Sjögren's syndrome, the immunomodulatory agent is a PDE4 inhibitor,
f. diabetes type-1, the immunomodulatory agent is a PDE4 inhibitor,
g. Crohn's disease and ulcerative colitis, the immunomodulatory agent is any one of TNF-alpha inhibitor, or PDE4 inhibitor,
h. psoriatic arthritis, the immunomodulatory agent is any one of IL-17 inhibitor, TNF-alpha inhibitor, or PDE4 inhibitor,
i. systemic lupus erythematosus (SLE), the immunomodulatory agent is a PDE4 inhibitor,
j. uveitis, the immunomodulatory agent is any one of TNF-alpha inhibitor, or PDE4 inhibitor,
k. neuro sarcoidosis, the immunomodulatory agent is a TNF-alpha inhibitor.

In preferred embodiments, selected immunomodulatory agents as summarized in Table 1 are used for the indicated medical indications in combination with any Treg cell activator such as GP120 or anti-CD4 antibody:

**Table 1:**

| Psoriasis | |
|---|---|
| TNF-alpha Inhibitors | Adalimumab (mAb), Etanercept (Fusion protein TNFR-Ig), Infliximab (mAb) |
| IL-23 Inhibitors | Risankizumab (mAb), Ustekinumab (mAb IL23/12), Guselkumab (mAb) |
| IL-17 Inhibitors | Secukinumab (mAb IL17A), Ixekizumab (mAb IL17A), Bimekizumab (mAb IL17A/F), Brodalumab (mAb IL17 RA) |
| PDE4 Inhibitors | Nerandomilast, Apremilast, Roflumilast |
| Interferon | Interferon-beta-1a, Interferon-beta-1b |
| Fumarate Immunomodulators Antimetabolite | dimethyl fumarate, diroximel fumarate, monomethyl fumarate |
| Immunosuppressants | Methotrexate |
| Retinoids | Acitretin |

| Rheumatoid Arthritis | |
|---|---|
| TNF-alpha Inhibitors | Adalimumab (mAb), Etanercept (Fusion protein TNFR-IgG), Infliximab (mAb) |
| IL-6R Inhibitors | Tocilizumab (mAb, IL6R), Sarilumab (mAb, IL6R) |
| CD20 Inhibitors Antimetabolite | Rituximab (mAb), Ocrelizumab (mAb), Ofatumumab (mAb) |
| Immunosuppresants | Methotrexate |
| CD80/86 Inhibitors | Abatacept (CTLA-4Ig) |
| PDE4 Inhibitors | Nerandomilast, Apremilast, Roflumilast |

| Sjögren's Syndrome | |
|---|---|
| CD40 Inhibitors | Frexalimab (mAb Anti-CD40L), Dazodalibep (fusion protein) |
| CD20 Inhibitors | Rituximab (mAb), Ocrelizumab (mAb), Ofatumumab (mAb) |
| Antimetabolite Immunosuppresants | Methotrexate |
| Antimalarials | Hydroxychloroquine |
| PDE4 Inhibitors | Nerandomilast, Apremilast, Roflumilast |

| Diabetes Type-1 | |
|---|---|
| CD80/86 Inhibitors | Abatacept (CTLA-4Ig) |
| CD20 Inhibitors | Rituximab (mAb), Ocrelizumab (mAb), Ofatumumab (mAb) |
| Interleukin-2 | Aldesleukin |
| PDE4 Inhibitors | Nerandomilast, Apremilast, Roflumilast |

| Crohn's Disease and Ulcerative Colitis | |
|---|---|
| TNF-alpha Inhibitors | Adalimumab (mAb), Etanercept (Fusion protein TNFR-Ig), Infliximab (mAb) |
| IL-23 Inhibitors | Risankizumab (mAb), Ustekinumab (mAb IL23/12), Guselkumab (mAb) |
| Integrin Blockers Antimetabolite | Vedolizumab (mAb alpha4β7 integrin) |
| Immunosuppressants | Methotrexate |
| PDE4 Inhibitors | Nerandomilast, Apremilast, Roflumilast |

| Multiple Sclerosis | |
|---|---|
| IL-17 Inhibitors | Secukinumab (mAb IL17A), Ixekizumab (mAb IL17A), Bimekizumab (mAb IL17A/F), Brodalumab (mAb IL17 RA) |
| Fumarate Immunomodulators | dimethyl fumarate, diroximel fumarate, monomethyl fumarate |
| Sphingosine 1-phosphate Receptor Modulator | Fingolimod, Ozanimod, Ponesimod, Siponimod small molecules |
| CD20 Inhibitors | Rituximab (mAb), Ocrelizumab (mAb), Ofatumumab (mAb) |
| IL-6R Inhibitors | Tocilizumab (mAb, IL6R), Sarilumab (mAb, IL6R) |
| TNF-alpha Inhibitors | Adalimumab (mAb), Etanercept (fusion protein TNFR-Ig), Infliximab (mAb) |
| PDE4 Inhibitors | Nerandomilast, Apremilast, Roflumilast |
| Lymphocyte-Depleting Biologic | Alemtuzumab (cytolytic mAb, anti-CD52) |
| Interferon | Interferon-beta-1a, Interferon-beta-1b |
| Copolymers Immunomodulators | Glatiramer acetate |
| CD40 Inhibitors | Frexalimab (mAb Anti-CD40L), Dazodalibep (fusion protein) |
| Pyrimidine Synthesis Inhibitors | Vidofludimus Calcium |

| Psoriatic Arthritis | |
|---|---|
| IL-17 Inhibitors | Secukinumab (mAb IL17A), Ixekizumab (mAb IL17A), Bimekizumab (mAb IL17A/F), Brodalumab (mAb IL17 RA) |
| IL-23 Inhibitors | Risankizumab (mAb), Ustekinumab (mAb IL23/12), Guselkumab (mAb) |
| Antimetabolite | |
| Immunosuppressants | Methotrexate |
| TNF-alpha Inhibitors | Adalimumab (mAb), Etanercept (Fusion protein TNFR-IgG), Infliximab (mAb) |
| PDE4 Inhibitors | Nerandomilast, Apremilast, Roflumilast |

| Systemic Lupus Erythematosus | |
|---|---|
| CD20 Inhibitors | Rituximab (mAb), Ocrelizumab (mAb), Ofatumumab (mAb) |
| Antimalarials | Hydroxychloroquine |
| B cell Inhibitors | Belimumab (mAb BAFF/BLyS) |
| IFN Type I Antagonists | Anifrolumab (mAb IFNAR) |
| Complement Inhibitors | Eculizumab (mAb C5) |
| PDE4 Inhibitors Antimetabolite | Nerandomilast, Apremilast, Roflumilast |
| Immunosuppressants | Methotrexate |

| Uveitis | |
|---|---|
| TNF-alpha Inhibitors | Adalimumab (mAb), Etanercept (Fusion protein TNFR-Ig), Infliximab (mAb), Certolizumab Pegol (mAb-PEG TNFR) |
| Antimetabolite Immunosuppressants | Methotrexate |
| PDE4 Inhibitors | Nerandomilast, Apremilast, Roflumilast |

| Systemic Sclerosis | |
|---|---|
| Antimetabolite | |
| Immunosuppressants | Methotrexate |
| IL-6R Inhibitors | Tocilizumab (mAb, IL6R), Sarilumab (mAb, IL6R) |
| PDE4 Inhibitors | Nerandomilast, Apremilast, Roflumilast |
| TNF-alpha Inhibitors | Adalimumab (mAb), Etanercept (Fusion protein TNFR-Ig), Infliximab (mAb) |

| Neuro-Sarcoidosis | |
|---|---|
| TNF-alpha Inhibitors | Adalimumab (mAb), Etanercept (Fusion protein TNFR-Ig), Infliximab (mAb) |
| Antimalarials | Hydroxychloroquine |

Table 1 summarizes possible selections of immunomodulatory agents together with a Treg activator (such as anti-CD4 antibody or GP120). The invention also comprises the selection of more than one immunomodulatory agent for a combination therapy with a Treg activator for the treatment of the indicated autoimmune or autoinflammatory diseases.

For example, for the treatment of psoriasis, the use of any one of TNF-alpha Inhibitors such as Adalimumab (mAb), Etanercept (Fusion protein TNFR-Ig), Infliximab (mAb); IL 23 Inhibitors such as Risankizumab (mAb), Ustekinumab (mAb IL23/12), Guselkumab (mAb); IL 17 Inhibitors such as Secukinumab (mAb IL17A), Ixekizumab (mAb IL17A), Bimekizumab (mAb IL17A/F), Brodalumab (mAb IL17 RA); PDE4 Inhibitors such as Nerandomilast, Apremilast, Roflumilast; Interferon such as Interferon-beta-1a, Interferon-beta-1b; Fumarate Immunomodulators such as dimethyl fumarate, diroximel fumarate, monomethyl fumarate; antimetabolite Immunosuppressants such as Methotrexate; Retinoids such as Acitretin in combination with a Treg activator such as GP120, a biologically active fragment or modified version thereof is preferred.

As a further example, for the treatment of multiple sclerosis, the use of any one of IL 17 Inhibitors such as Secukinumab (mAb IL17A), Ixekizumab (mAb IL17A), Bimekizumab (mAb IL17A/F), Brodalumab (mAb IL17 RA); Fumarate Immunomodulators such as dimethyl fumarate, diroximel fumarate, monomethyl fumarate; Sphingosine 1-phosphate Receptor Modulator such as Fingolimod, Ozanimod, Ponesimod, Siponimod small molecules; CD20 Inhibitors such as Rituximab (mAb), Ocrelizumab (mAb), Ofatumumab (mAb); IL-6R Inhibitors such as Tocilizumab (mAb, IL6R), Sarilumab (mAb, IL6R); TNF-alpha Inhibitors such as Adalimumab (mAb), Etanercept (fusion protein TNFR-Ig), Infliximab (mAb); PDE4 Inhibitors such as Nerandomilast, Apremilast, Roflumilast; Lymphocyte-Depleting Biologic such as Alemtuzumab (cytolytic mAb, anti-CD52); Interferon such as Interferon-beta-1a, Interferon-beta-1b; Copolymers Immunomodulators such as Glatiramer acetate; CD40 Inhibitors such as Frexalimab (mAb Anti-CD40L), Dazodalibep (fusion protein);
Pyrimidine Synthesis Inhibitors such as Vidofludimus Calcium in combination with a Treg activator such as GP120, a biologically active fragment or modified version thereof is preferred.

As a further example, for the treatment of rheumatoid arthritis, the use of any one of TNF-alpha Inhibitors such as Adalimumab (mAb), Etanercept (Fusion protein TNFR-IgG), Infliximab (mAb); IL-6R Inhibitors such as Tocilizumab (mAb, IL6R), Sarilumab (mAb, IL6R); CD20 Inhibitors such as Rituximab (mAb), Ocrelizumab (mAb), Ofatumumab (mAb); antimetabolite Immunosuppressants such as Methotrexate; CD80/86 Inhibitors such as Abatacept (CTLA-4Ig); PDE4 Inhibitors such as Nerandomilast, Apremilast, Roflumilast in combination with a Treg activator such as GP120, a biologically active fragment or modified version thereof is preferred.

As a further example, for the treatment of systemic sclerosis, the use of any one of antimetabolite Immunosuppressants such as Methotrexate; IL-6R Inhibitors such as Tocilizumab (mAb, IL6R), Sarilumab (mAb, IL6R), PDE4 Inhibitors such as Nerandomilast, Apremilast, Roflumilast; TNF-alpha Inhibitors such as Adalimumab (mAb), Etanercept (Fusion protein TNFR-Ig), Infliximab (mAb) in combination with a Treg activator such as GP120, a biologically active fragment or modified version thereof is preferred.

Interestingly, despite the distinct molecular interactions (mechanism of action), the immunomodulatory agents have a common mode of action e.g., ameliorating the cellular activation and/or proliferation of reactive immune cells and reduction of pro-inflammatory cytokines or mechanisms. Since these inflammatory processes are characteristic for autoimmune and autoinflammatory diseases, these agents are suitable for use in various autoimmune or autoinflammatory diseases. This transferability of agents having a common mode of action is also evident when comparing the standard therapies for autoimmune or autoinflammatory diseases. Many agents such as TNF inhibitors are used in the context of different autoimmune or autoinflammatory diseases, including, but limited to psoriasis, rheumatoid arthritis, Crohn's disease and ulcerative colitis multiple sclerosis.

This transferability of agents is also shown by the data presented herein. The panel of agents applied in this patent application have different mechanisms of action, but a common mode of action and subsequently show synergistic efficacy on T cells from healthy donors and autoimmune or autoinflammatory patients. Interestingly, the data show that some agents only become effective in additional diseases through the combination with Treg activating agents.

The present invention also relates to pharmaceutical compositions, comprising an immunologically effective combination as defined herein and a pharmaceutically acceptable carrier or diluent.

In one aspect, the pharmaceutically composition comprises an immunologically effective combination as defined herein and a pharmaceutically acceptable carrier or diluent for the use in the prevention or treatment of an autoinflammatory disease.

In a preferred embodiment, the immunomodulatory agent and the Treg activator in the immunologically effective combination are provided in a single dose form or in two distinct dose forms. The one embodiment, the single dose form contains both the immunomodulatory agent and the Treg activator. In an alternative embodiment, one dose form contains the immunomodulatory agent and another dose form contains the Treg activator.

The pharmaceutical composition may be formulated with any known pharmaceutically acceptable carrier or diluent as well as any other known adjuvants and excipients in accordance with conventional techniques. The pharmaceutically acceptable carriers, diluents, adjuvants and excipients should be suitable for the chosen inductor of the present invention and the chosen mode of administration. A pharmaceutical composition of the present invention may also include diluents, fillers, salts, buffers, detergents (e. g., a nonionic detergent), stabilizers (e. g., sugars or protein-free amino acids), preservatives, tissue fixatives, solubilizers, and/or other materials suitable for inclusion in a pharmaceutical composition.

The pharmaceutical compositions of the present invention can be formulated by methods known to those skilled in the art. For example, such pharmaceutical compositions can be used parenterally, as injections which are sterile solutions or suspensions including the compositions along with water or another pharmaceutically acceptable liquid. For example, such compositions may be formulated as unit doses that meet the requirements for the preparation of pharmaceuticals by appropriately combining the compositions with pharmaceutically acceptable carriers, diluents, adjuvants or excipients, specifically with sterile water, physiological saline, a vegetable oil, emulsifier, suspension, surfactant, stabilizer, flavoring agent, excipient, vehicle, preservative, binder or such. In such preparations, the amount of active ingredient is adjusted.

The two immunologically active components of the combination of the present invention, i.e. the immunomodulatory agent and the Treg activator can be combined in a single composition for use in the treatment or prevention of an autoinflammatory disease. In alternative embodiments, the immunomodulatory agent and the Treg activator are provided separately in two distinct compositions.

In a first aspect, the composition containing the immunomodulatory agent and the composition containing the Treg activator can be combined prior to administration to a subject in need thereof. In a second aspect, the composition comprising the immunomodulatory agent and the composition comprising the Treg activator are administered as a single dose or repeated dose to a subject in need thereof. In a third aspect, the composition comprising the immunomodulatory agent and the composition comprising the Treg activator are administered to a subject in need thereof within a desired time interval. For example, the composition comprising the immunomodulatory agent is given to a subject in need thereof at a first time point, while the composition comprising the Treg activator is given to a subject in need thereof at a second time point within a given time interval. It is the aim to have no or only little Treg resistance, so it is important to suppress inflammation sufficiently by the pre-treatment with the immunoregulatory agent before administration of the Treg activator as immunoregulatory agent. Preferably, the time interval for administering a single or repeated dose of the first composition comprising the immunomodulatory agent and administering a single or repeated dose of the second composition comprising the Treg activator is at least 7 days, preferably at least 14 days, more preferably at least 30 days. In more preferred embodiments, the time interval for administering a single or repeated dose of the first composition comprising the immunomodulatory agent and administering a single or repeated dose of the second composition comprising the Treg activator is at least 4 weeks, preferably between 4 weeks and 6 weeks. The administration of the first and/or second composition can be repeated several times. In addition, the number of administrations, the composition and concentration of the compositions comprising the immunomodulatory agent and/or Treg activator can vary. For example, the individual doses given to a subject may vary and depend, inter alia, from gender, age, constitution and state of illness.

In some embodiments, in the pharmaceutical composition of any one of embodiments disclosed herein, the immunomodulatory agent is in the same dosage form as the Treg activator such that the immunomodulatory agent is administered concurrently with the Treg activator. In a preferred embodiment, the dosage form is a single fluid. In some embodiments, the dosage form including the immunomodulatory agent and the Treg activator is prepared by combining a composition including the immunomodulatory agent and a composition including the Treg activator. In some embodiments, in the pharmaceutical composition of any one of embodiments disclosed herein the immunomodulatory agent is in a separate dosage form from the Treg activator such that the immunomodulatory agent can be administered before or after the Treg activator. A delayed administration of the Treg activator relative to the immunomodulatory agent is preferred.

The invention also relates to the use of an immunologically effective combination comprising at least one immunomodulatory agent and an CD4-targeting activator of regulatory T cells (Treg activator) for the manufacturing of a medicament that promotes and restores immune tolerance in a human or animal subject. The manufacturing process can involve the preparation of a first dosage form, in which the immunomodulatory agent is provided, and the preparation of a second dosage form, in which the Treg activator is provided, while both dosage forms are prepared to be administered to a subject in need thereof.

### Brief Descriptions of the Drawings:

**Figure 1****:** Synergistic effects of GP120 with different modulators on proliferation of T cells from healthy controls.
**Figure 2****:** Synergistic effects of GP120 with different modulators on proliferation of T cells from patients with multiple sclerosis.
**Figure 3****:** Synergistic effects of GP120 with different modulators on proliferation of T cells from patients with psoriasis.
**Figure 4****:** Synergistic effects of GP120 with different modulators on proliferation of T cells from patients with systemic sclerosis.

### Examples:

The present invention is further illustrated in the following examples. By no means shall the present invention be restricted to these specific examples. Also, the combination of features or even embodiments are encompassed by the spirit of the present invention.

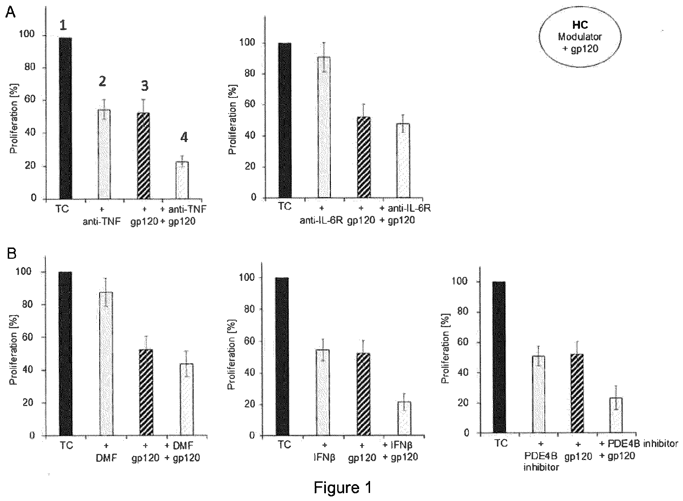

**Figure 1** shows the synergistic effects of GP120 with various immunomodulatory agents on proliferation of T cells from healthy controls.

T cells from healthy donors (HC) cocultured with allogeneic dendritic cells (mixed lymphocyte reaction) in the absence or presence of 1 µg/ml GP120 are shown. Different modulators were added to these cultures. A) From left to right: anti-human TNF-alpha mAb (50 ng/ml), anti-human IL-6R mAb (30 ng/ml). B) From left to right: Dimethyl fumarate (DMF, 2 µM), recombinant human IFN-beta (1 IU/ml), PDE4B inhibitor (2 µM). On day 4 of culture, 37 kBq [³H] Thymidine (³H-Tdr) was added to each well and cells were cultured for an additional 16 h. T cell proliferation was measured by ³H-Tdr incorporation using a liquid β-scintillation counter. Bars show mean proliferation (in %) ± SEM of n=5 individual experiments.

Column 1 is a positive control and equals to the optimal proliferation of T cells from healthy doners, stimulated with allogenic dendritic cells (DC). The effect of the immunomodulatory agent DMD and/or the Treg activator GP120 is shown as reduced proliferation in % of optimal proliferation. No modulating effect means that there is no reduced proliferation of T cells.

Column 2 shows the T cell modulating effect of the immunomodulatory agent DMD as evident from the reduced proliferation of T cells.

Column 3 shows the T cell modulating effect of the Treg activator GP120 as evident from the reduced proliferation of T cells.

Column 4 shows the synergistic effect of the immunomodulatory agent DMD and the Treg activator GP120 on T cell activity, which only occurs in the presence of both components. A synergistic effect can also be postulated if no effect on T cell proliferation was measured in the presence of only one component (i.e., DMD or GP120). This can be explained in that both components use different surface markers and signaling pathways on the T cells (e.g., TNF receptor and CD4), which independently trigger a signaling cascade. While a single component may be too weak to measurably modulate T cells, the addition of both effects still leads to a stronger modulation than would be measurable with the 2nd component alone.

Figure 1A on the left shows that the optimal proliferation of T cells is reduced by approximately 50% by blocking TNF signal transduction (anti-TNF, gray bar). The reduction by GP120 is even greater. The synergistic effect of anti-TNF and GP120 leads to a significantly greater suppression of T cells of up to 80%. The data show that anti-TNF and GP120 synergistically inhibit the activation of T cells. Figure 1A on the right shows the influence of anti-IL6R and GP120 on the proliferation of activated T cells. In contrast to GP120, anti-IL-6R can only slightly suppress T cell proliferation. Both factors together show a synergistic effect and suppress T cell proliferation more strongly than either factor alone.

Figure 1B on the left shows that, in contrast to GP120, DMF has only a weak inhibitory effect on the activation of T cells (approx. 15% for DMF). However, it is observed that DMF in combination with GP120 suppresses significantly more than DMF or GP120 alone. Here, too, a synergistic effect of DMF and GP120 is postulated. Figure 1B middle shows that GP120 also works synergistically with IFN-beta and inhibits the proliferation of T cells, because of T cell stimulation, significantly more than either factor can achieve alone. Figure 1B right shows that GP120 also inhibits the activation of T cells synergistically with PDE4B inhibitors. In combination (PBE4B inhibitor to plus GP120), proliferation is suppressed by up to 80%, a value that cannot be achieved by one factor alone.

In summary, the data in Figure 1 show that GP120 can inhibit the activation of T cells synergistically with a variety of biologics that modulate T cell activation in different cellular signaling pathways.

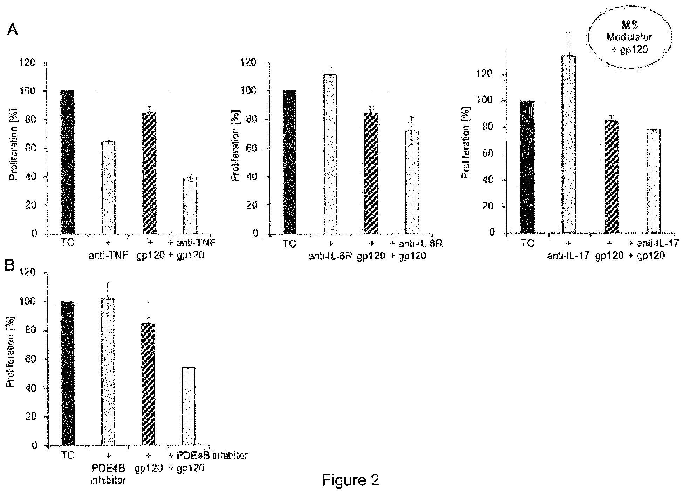

**Figure 2** shows the synergistic effects of GP120 with various immunomodulatory agents in the suppression of T cells from the blood of MS patients. The synergistic effect (GP120 plus biologics) is evident for all four biologics to varying degrees.

T cells from multiple sclerosis patients (MS) were cocultured with allogeneic dendritic cells (mixed lymphocyte reaction) in the absence or presence of 1 µg/ml GP120. Different modulators were added to these cultures. A) From left to right: anti-human TNF-alpha mAb (50 ng/ml), anti-human IL-6R mAb (30 ng/ml), anti-human IL-17A mAb (50 ng/ml). B) PDE4B inhibitor (2 µM).

On day 4 of culture, 37 kBq [³H] Thymidine (³H-Tdr) was added to each well and cells were cultured for an additional 16 h. T cell proliferation was measured by ³H-Tdr incorporation using a liquid beta-scintillation counter. Bars show mean proliferation (in %) ± SEM of n=2 individual experiments.

GP120 acts synergistically with anti-TNF, anti-IL-6R, anti-IL-17 and PDE4B inhibitors in the suppression of T cell activity from the blood of patients.

The marginal effect of the biologics alone and the reduced effect of GP120 alone compared to HC as shown in Figure 1 is due to the dysregulated activity of T cells from MS patients described in the literature. In this case, the synergistic effect of the biologics with GP120 appears to be particularly valuable for the therapeutic effect of the combination compared to the individual components. Figure 2 shows again that GP120 acts synergistically with a variety of biologics that block different signaling pathways in T cells and significantly increases the desired outcome (reduced proliferation).

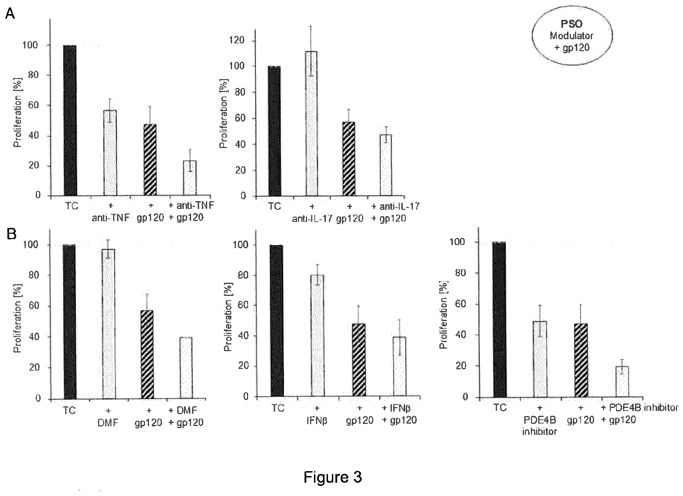

**Figure 3** shows the influence of various immunomodulatory agents and the Treg activator GP120 on the proliferation of T cells isolated from the blood of psoriasis patients.

T cells from psoriasis patients (PSO) were co-cultured with allogeneic dendritic cells (mixed lymphocyte reaction) in the absence or presence of 1 µg/ml GP120. Additionally, different modulators were added to these cultures. A) From left to right: anti-human TNF-alpha mAb (50 ng/ml), anti-human IL-17A mAb (50 ng/ml). B) From left to right: Dimethyl fumarate (DMF, 2 µM), recombinant human IFN-beta (1 IU/ml), PDE4B inhibitor (2 µM).

On day 4 of culture, 37 kBq [³H] Thymidine (³H-Tdr) was added to each well and cells were cultured for an additional 16 h. T cell proliferation was measured by ³H-Tdr incorporation using a liquid β-scintillation counter. Bars show mean proliferation (in %) ± SEM of n=3 individual experiments.

While anti-TNF, the PDE4B inhibitor and GP120 significantly suppress the proliferation of T cells, this modulating effect is only slight for IFN-beta and not detectable for anti-IL-17 and DMF. However, all biologics, including anti-IL-17 and DMF, show a synergistic suppressive effect in combination with GP120.

The data again show that the combination with GP120 has a synergistic effect in suppressing T cell activity, even if the biologic alone (here anti-IL-17 and DMF) is not potent enough to efficiently inhibit the proliferation of T cells.

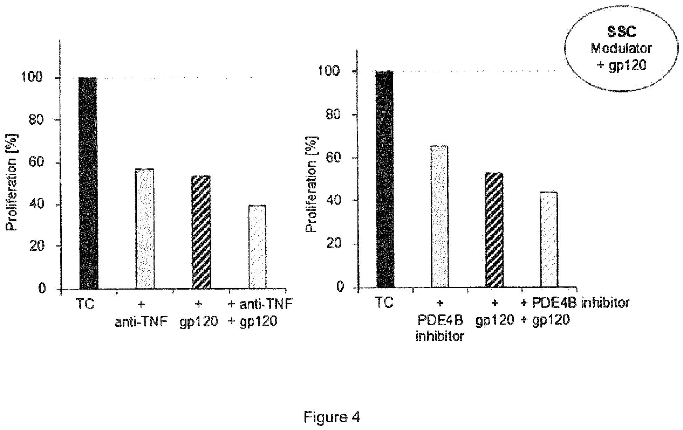

**Figure 4** shows the synergistic effects of GP120 with various immunomodulatory agents on proliferation of T cells from patients with systemic sclerosis.

T cells from systemic sclerosis patients (SSC) were cocultured with allogeneic dendritic cells (mixed lymphocyte reaction) in the absence or presence of 1 µg/ml GP120. Different modulators were added to these cultures: anti-human TNF-alpha mAb (50 ng/ml), PDE4B inhibitor (2 µM).

On day 4 of culture, 37 kBq [³H] Thymidine (³H-Tdr) was added to each well and cells were cultured for an additional 16 h. T cell proliferation was measured by ³H-Tdr incorporation using a liquid beta-scintillation counter. Bars show proliferation in %.

All three factors alone show an inhibitory effect on proliferation. The synergistic effect of anti-TNF + GP120 and PDE4B inhibitor + GP120 is in every case higher than with one of the three factors alone.

In summary, the data show that the combination of GP120 and immunomodulatory agents synergistically increases the effect of various single immunomodulatory agents used to treat excessive immune responses involving T cells. This synergistic effect is not toxic and has no measurable influence on the viability of the stimulated T cells. In its ability to suppress the activation of T cells, GP120 shows synergistic effects with immunomodulatory agents such as anti-TNF, anti-IL6R, DMF, IFN-beta and PDE4B inhibitors. These biologics intervene in different, independent signaling pathways. One mechanism that the immunomodulatory agents have in common is that they intervene in signaling pathways that are important for the activation of T cells. The immunomodulatory agents thus exert their effect by suppressing individual signaling pathways for T cell activation. GP120 can synergistically enhance the inhibition of all these signaling pathways and thus inhibit the activation of T cells to a greater extent.

The data shown in Figures 2 to 4 demonstrate the synergistic effects of immunomodulatory agents in combination with the Treg activator GP120 on proliferation of T cells from patients suffering of multiple sclerosis (Fig. 2), psoriasis (Fig. 3) or systemic sclerosis (Fig. 4). The data show that GP120 can be used as an enhancer of the mechanistic effects of many biologics in different indications. The decisive factor for this synergistic effect is an excessive immune reaction involving T cells. This applies multiple sclerosis (MS), psoriasis (PSO), sclerosis (SSC), and a variety of other autoinflammatory diseases and autoimmune diseases listed in Table 1. The data also show that a combined application of an immunomodulatory agent and a Treg activator is superior to any monotherapy in the treatment of autoimmune disease.

### Materials and Methods

### Isolation and culture of human immune cells

PBMC from either multiple sclerosis (MS) patients, psoriasis (PSO) patients, patients with systemic sclerosis (SSC), or healthy donors (HC) were isolated from peripheral blood within 16 h after blood collection using density gradient centrifugation. Blood was kept at room temperature before PBMC enrichment. After isolation, human cells were cultured in X-VIVO-15 (Lonza, Belgium).

### Generation of dendritic cells

Dendritic cells (DC) were generated from isolated PBMC from the peripheral blood of healthy donors. 10-15x10⁶ PBMC (per well) were seeded in a 6-well culture plate in 2 ml RPMI + 1.5% heat-inactivated plasma and incubated for 30 minutes in an incubator at 37 °C and 5% CO₂. During this incubation time, monocytes adhered to the plastic surface. The non-adherent cells were washed off several times with 1 ml pre-warmed PBS and the purity of the monocyte culture was visually checked under a light microscope. Afterwards monocytes were cultured in X-VIVO-15 (3 ml/well) supplemented with 1% heat-inactivated plasma + 400 IU/ml rh GM-CSF (Sargramostim / Leukine) + 200 IU/ml rh IL-4 (Immunotools) for 6 days. On days 2 and 4 of the culture, cells were fed by removing 1 ml of medium per well and replacing it with 1 ml of culture medium supplemented with 800 IU/ml rh GM-CSF. DC were stored frozen in aliquots until use.

### Isolation and CFSE labeling of CD3⁺ T cells

Untouched CD3⁺ T cells were isolated using Pan T cell isolation kit (Miltenyi Biotec) according to manufacturer's instructions. The purity of the isolated CD3⁺ T cells was checked by flow cytometry. For this purpose, cells were stained with the following antibodies: anti-human CD3 FITC (UCHT1), anti-human CD4 PE-Cy7 (RPA-T4), anti-human CD8 APC (RPA-T8), all from BD Pharmingen. Stained cells were measured on LSRII with FACS Diva Software (BD Bioscience) and analyzed using FlowJo software.

In some experiments, isolated CD3⁺ T cells from healthy donors were stained with the proliferation dye CFSE. Therefore, CD3⁺ T cells were resuspended in pre-warmed PBS to a final concentration of 2x10⁷/ml and stained with 1 µM CFSE for 20 minutes at 37 °C in the dark. Afterwards, cells were washed twice with X-VIVO-15/10% HSA and further stored at 37 °C until use in X-VIVO-15.

### Mixed leukocyte reaction

A mixed leukocyte reaction served as the basis for the investigations. For this purpose, CD3⁺ T cells from healthy donors, MS patients, PSO patients or SSC patients were co-cultured with allogeneic DC (TC:DC ratio of 20:1) in the absence or presence of GP120 (ActiTrexx GmbH / Polymun Scientific GmbH) (1 µg/ml). In further approaches, different modulators were added to these cultures to investigate possible synergistic effects on T cell proliferation. The following modulators were used so far (Table 2):

**Table 2:**

| **Modulator** | **Supplier** | **Concentration** |
|---|---|---|
| Tocilizumab (RoActemra^{®}), anti-human IL6R mAb | Roche | 30 ng/ml |
| Ixekizumab (Taltz^{®}), anti-human IL-17A mAb | Eli Lilly | 50 ng/ml |
| Adalimumab (Amgevita^{®}), anti-human TNF-alpha mAb | Amgen | 50 ng/ml |
| rh IFN-beta-1a | Miltenyi Biotec | 1 IU/ml |
| Nerandomilast, PDE4B inhibitor | Hycultec | 2 µM |
| Dimethyl fumarate | Merck | 2 µM |

### Measurement of CD3⁺ T cell Proliferation

T cell proliferation was analyzed using two methods equally suitable for evaluating the experiments:
a) Incorporation of ³H-Tdr:
   On day 4 of culture, ³H-Tdr was added to each well (37 kBq/well) and cells were cultured for an additional 16 h in an incubator at 37 °C and 5% CO₂. CD3⁺ T cell proliferation was measured by ³H-Tdr incorporation using a liquid beta-scintillation counter.
b) Flow cytometric analysis of CFSE-labeled CD3⁺ T cells:
   Some experiments were performed using CFSE-labeled CD3⁺ T cells. On day 6 of culture, cells were harvested and stained with the viability dye Zombie NIR (Biolegend) and anti-human CD4 PE-Cy7 (RPA-T4) antibody (BD Pharmingen). Stained cells were measured on LSRII with FACS Diva Software (BD Bioscience) and analyzed using FlowJo software. The viability dye Zombie NIR enabled the distinction between live and dead cells, while CD4 staining differentiated CD4⁺ T cells from CD4⁻ T cells (equivalent to CD8⁺ T cells). The CFSE signal was assessed within the viable CD4⁺ and CD4⁻ T cell populations.
Non-proliferating cells exhibited a strong CFSE signal. With each cell division, the CFSE fluorescence intensity was progressively halved in the daughter cells. Thus, the gradual reduction of the CFSE signal served as an indicator of T cell proliferation.

### References:

1. Protection from graft-versus-host disease by HIV-1 envelope protein gp120-mediated activation of human CD4+CD25+ regulatory T cells. Becker C, Taube C, Bopp T, Becker C, Michel K, Kubach J, Reuter S, Dehzad N, Neurath MF, Reifenberg K, Schneider FJ, Schmitt E, Jonuleit H. Blood. 2009 Aug 6;114(6):1263-9. doi: 10.1182/blood-2009-02-206730. Epub 2009 May 13.
2. Interferon-Beta Therapy of Multiple Sclerosis Patients Improves the Responsiveness of T Cells for Immune Suppression by Regulatory T Cells. Trinschek B, Luessi F, Gross CC, Wiendl H, Jonuleit H. Int J Mol Sci. 2015 Jul 17;16(7):16330-46. doi: 10.3390/ijms160716330.
3. Dimethyl Fumarate Therapy Significantly Improves the Responsiveness of T Cells in Multiple Sclerosis Patients for Immunoregulation by Regulatory T Cells. Schlöder J, Berges C, Luessi F, Jonuleit H. Int J Mol Sci. 2017 Jan 28;18(2):271. doi: 10.3390/ijms18020271.
4. Pro-inflammatory cytokines and prostaglandins induce maturation of potent immunostimulatory dendritic cells under fetal calf serum-free conditions. Jonuleit H, Kühn U, Müller G, Steinbrink K, Paragnik L, Schmitt E, Knop J, Enk AH. Eur J Immunol. 1997 Dec;27(12):3135-42. doi: 10.1002/eji.1830271209.

## Claims

1. An immunologically effective combination, comprising at least one immunomodulatory agent and an CD4-targeting activator of regulatory T cells (Treg activator) for use in the prevention or treatment of an autoinflammatory disease.

2. The immunologically effective combination for the use according to claim 1, wherein said
a. immunomodulatory agent modulates, suppresses or modifies immune responses in a subject without specifically targeting immune tolerance mechanisms,
b. Treg activator promotes and restores immune tolerance by activating Treg and thereby suppressing autoinflammatory T effector cells.

3. The immunologically effective combination for the use according to claim 1 or claim 2, wherein said immunomodulatory agent is selected from the group consisting of
a. IL-6R inhibitors, IL-17 inhibitors, IL-23 inhibitors, TNF- alpha inhibitors, PDE4 inhibitors, interferon, fumarate immunomodulators, and/or
b. methotrexate, retinoids, IL-2, CD20 inhibitors, CD80/86 inhibitors, hydroxychloroquine, integrin blockers, sphingosine 1-phosphate receptor modulator, lymphocyte-depleting agents, glatiramer acetate, CD40 inhibitors, preferably CD40L blockers, pyrimidine synthesis inhibitors, B cell inhibitors.

4. The immunologically effective combination for the use according to claim 3, wherein said
a. IL-6R inhibitor is an anti-IL6R antibody,
b. IL-17 inhibitor is an anti-IL17RA antibody or an anti-IL17A antibody,
c. IL-23 inhibitor is an anti-IL23R antibody,
d. TNF- alpha inhibitor is an anti-TNF- alpha antibody, or a TNFR-IgG fusion protein,
e. PDE4 inhibitor is an PDE4B inhibitor,
f. interferon is interferon-beta-1a or interferon-beta-1b,
g. fumarate immunomodulator is dimethyl fumarate (DMF), diroximel fumarate or monomethyl fumarate.

5. The immunologically effective combination for the use according to claim 3, wherein said IL-6R inhibitor is Tocilizumab, or Sarilumab,
said IL-17 inhibitor is Ixekizumab, Secukinumab, Bimekizumab, or Brodalumab,
said IL-23 inhibitor is Risankizumab, Ustekinumab, or Guselkumab,
said TNF-alpha inhibitor is Adalimumab, Etanercept, or Infliximab,
said PDE4 inhibitor is Nerandomilast, Apremilast, or Roflumilast said interferon is interferon-beta-1a,
said fumarate immunomodulator is DMF.

6. The immunologically effective combination for the use according to claim 3, wherein said CD20 inhibitor is Rituximab, Ocrelizumab, Ublituximab, or Ofatumumab,
said CD80/86 inhibitor is Abatacept,
said integrin blocker is Vedolizumab,
said sphingosine 1-phosphate receptor modulator is Figolimod, Ozanimod, Ponesimod, or Siponimod,
said lymphocyte-depleting agent is Alemtuzumab,
said CD40L blocker is Frexalimab or Dazodalibep,
said pyrimidine synthesis inhibitor is Vidofludimus Calcium,
said IL-2 is Aldesleukin,
said B cell inhibitor is Belimumab.

7. The immunologically effective combination for the use according to any one of claims 1 to 6, wherein the Treg activator is any one of anti-CD4 antibodies or functional fragments thereof, or GP120 (HIV-1 glycoprotein 120) or biologically active fragments thereof.

8. The immunologically effective combination for the use according to claim 7, wherein the anti-CD4 antibody is Tregalizumab or Palixizumab.

9. The immunologically effective combination for the use according to any one of claims 1 to 8, wherein the at least one immunomodulatory agent is provided in a first dosage form and the Treg activator is provided as a second dosage form, wherein a dosage regimen provides for the first dosage form to be administered to a subject concurrently with or prior to the second dosage form.

10. The immunologically effective combination for the use according to any one of claims 1 to 9, wherein the autoinflammatory disease is any one of psoriasis, rheumatoid arthritis, Sjögren's syndrome, diabetes type-1, Crohn's disease and ulcerative colitis, multiple sclerosis, psoriatic arthritis, systemic lupus erythematosus (SLE), uveitis, systemic sclerosis, neuro sarcoidosis.

11. The immunologically effective combination for the use according to claim 10, wherein for the treatment of
a. psoriasis, the immunomodulatory agent is any one of TNF-alpha inhibitor, IL-17 inhibitor, PDE4 inhibitor, interferon, or fumarate immunomodulator;
b. multiple sclerosis, the immunomodulatory agent is any one of fumarate immunomodulator, TNF-alpha inhibitor, IL-6R inhibitor, IL-17 inhibitor, PDE4 inhibitor, or interferon;
c. systemic sclerosis, the immunomodulatory agent is any one TNF-alpha inhibitor, IL-6R inhibitor, or PDE4 inhibitor;
d. rheumatoid arthritis, the immunomodulatory agent is any one TNF-alpha inhibitor, IL-6R inhibitor, or PDE4 inhibitor.
e. Sjögren's syndrome, the immunomodulatory agent is a PDE4 inhibitor,
f. diabetes type-1, the immunomodulatory agent is a PDE4 inhibitor,
g. Crohn's disease and ulcerative colitis, the immunomodulatory agent is any one of TNF-alpha inhibitor, or PDE4 inhibitor,
h. psoriatic arthritis, the immunomodulatory agent is any one of IL-17 inhibitor, TNF-alpha inhibitor, or PDE4 inhibitor,
i. systemic lupus erythematosus (SLE), the immunomodulatory agent is a PDE4 inhibitor,
j. uveitis, the immunomodulatory agent is any one of TNF-alpha inhibitor, or PDE4 inhibitor,
k. neuro sarcoidosis, the immunomodulatory agent is a TNF-alpha inhibitor.

12. A pharmaceutical composition, comprising an immunologically effective combination according to any one of claims 1 to 9 and a pharmaceutically acceptable carrier or diluent.

13. A pharmaceutical composition, comprising an immunologically effective combination according to any one of claims 1 to 9 and a pharmaceutically acceptable carrier or diluent for the use in the prevention or treatment of an autoimmune disease or autoinflammatory disease.

14. The pharmaceutical composition according to claim 12 or claim 13, wherein the immunomodulatory agent and the Treg activator in the immunologically effective combination are provided in a single dose form or in two distinct dose forms.

15. Use of an immunologically effective combination comprising at least one immunomodulatory agent and an CD4-targeting activator of regulatory T cells (Treg activator) according to any one of claims 1 to 9 for the manufacturing of a medicament that promotes and restores immune tolerance in a human or animal subject.
